# EUROPÄISCHE PATENTSCHRIFT

(11) **EP 1 557 138 B1**
(45) Veröffentlichungstag und Bekanntmachung des Hinweises auf die Patenterteilung: **05.12.2012**
(21) Anmeldenummer: 05001229.3
(22) Anmeldetag: 21.01.2005
(51) Int. Cl.: A61F 2/90

(54) **Expandierbarer Stent mit Koppeleinrichtung**
Expandable stent with coupling device
Stent expansible avec dispositif de couplage

(30) Priorität: 21.01.2004 DE 102004003093; 09.11.2004 EP 04026619
(43) Veröffentlichungstag der Anmeldung: 27.07.2005
(73) Patentinhaber: Admedes Schuessler GmbH, 75179 Pforzheim (DE)
(72) Erfinder: Scherrible, Frank, 75236 Kämpfelbach (DE)
(74) Vertreter: Müller-Boré & Partner Patentanwälte

(56) Entgegenhaltungen:
- DE-A1- 19 728 337
- DE-U1-202004 000 896
- US-A- 5 019 102
- US-A1- 2002 111 671
- US-A1- 2003 199 969
- US-B1- 6 245 102

## Beschreibung

Die Erfindung betrifft einen Stent zum Einsetzen und/oder Expandieren in einem Lumen bzw. zum Implantieren in einen lebenden Körper, mit mindestens zwei Abschnitten einer Stentstruktur, die beim Expandieren des Stents aufeinander zu bewegt werden.

Stents der oben genannten Art werden verwendet, um ein Lumen bzw. einen Kanal eines lebenden Körpers, wie beispielsweise Blutgefäße, Speiseröhre, Harnröhre oder Nierengänge, durch Expandieren einer im wesentlichen rohrförmigen Wandstruktur des Stents im Inneren des Kanals gegen Kollabieren oder Verschließen zu schützen. Der eingesetzte Stent kann dabei den Strömungsquerschnitt des Lumen für ein darin strömendes Medium vergrößern oder dauerhaft ausreichend groß halten. Ferner werden Stents als Träger von Medikamenten verwendet, die in einem Körperkanal eine zumindest lokale Therapie möglich machen. Stents können darüber hinaus als Aneurismen-Stent bzw. Endoprothese für intrazelebrale Gefäßaussackungen oder als intraluminaler Stent eingesetzt werden. Weiterhin werden Stents bzw. Spreizstrukturen als Träger für Implantate verwendet.

Die Wand- bzw. Stentstruktur solcher Stents weist eine Vielzahl von Stegen auf, die miteinander an Stegverbindern bzw. Knoten verbunden sind. Die Stege sind aus einem flexiblen Material, wie etwa Nitinol oder Edelstahl gestaltet, so dass der Stent insgesamt eine geringfügig flexible Wandstruktur aufweist. Es sind ferngesteuert zu expandierende Stents bekannt, die beispielsweise mittels einer Art Bowdenzug betätigt werden, oder selbstexpandierende Stents, die vorgespannt sind und ihren Durchmesser bei entsprechender Freigabe selbsttätig vergrößern.

Damit der gewünschte Strömungsquerschnitt des Lumens sichergestellt ist, ist es wünschenswert, dass ein einmal eingesetzter und expandierter Stent seine Form möglichst dauerhaft beibehält und nicht später weiter expandiert oder komprimiert. Ferner wäre es wünschenswert, wenn der Stent eine vordefinierte Form annehmen und diese dann beibehalten würde.

US2002/111671 A1 offenbart einen Verriegelungsstent mit einer verriegelbaren Stentzelle, die ein erstes und ein zweites Sperrelement aufweist. In der Verriegelungsposition im expandierten Zustand des Stents wird eine Stützfestigkeit aufgrund der verriegelten Stentzelle erhöht.

US2003/0199969 A1 offenbart einen expandierbaren Stent mit radialen Gleit- und Sperrelementen, die ein Expandieren des Stents ermöglichen, während ein Kollabieren eines expandierten Stents verhindert wird.

DE 197 28 337 A1 offenbart eine implantierbare Gefäßstütze Gemäß dem Oberbegraft von Anspruch 1 die biologisch abbaubar und auf unterschiedliche Durchmesser einstellbar ist.

Die Aufgabe der Erfindung besteht in der Schaffung eines Stents, bei dem unerwünschte Deformationen zuverlässig verhindert werden kann.

Diese Aufgabe wird durch den Stent gemäß Anspruch 1 gelöst. Bevorzugte Ausführungsformen der Erfindung sind Gegenstand der Unteransprüche.

Die Aufgabe ist erfindungsgemäß durch einen Stent. Trägerstruktur zum Einsetzen bzw. Einführen und/oder Expandieren bzw. Stützen bzw. Tragen in einem Lumen gelöst, mit mindestens zwei Abschnitten einer Stentstruktur, die beim Expandieren des Stents im wesentlichen aufeinander zu bewegt werden, bei dem zwischen den beiden Abschnitten mindestens eine Koppeleinrichtung angeordnet ist, mittels der nach zumindest einer vordefinierten Bewegung bzw. Wegstrecke der beiden Abschnitte diese Abschnitte miteinander mit den Merchemalen nach Anspruch 1 gelöst.

Erfindungsgemäß ist zwischen zwei Abschnitten des Stents bzw. der Spreiz- bzw. Trägerstruktur, welche sich bei einer Expansion des Stents bzw. der Spreiz- bzw. Trägerstruktur relativ zueinander bewegen eine Koppelung vorgesehen, welche die Abschnitte in ihrer versetzten Lage relativ zu einander fixiert und auf diese Weise eine undefinierte weitere Expansion und/oder Kompression des Stents nicht mehr zuläßt. Ferner nehmen die zu bewegenden Abschnitte des Stents aufgrund der erfindungsgemäßen Koppeleinrichtung eine vorbestimmte Lage relativ zueinander ein, so dass der Stent insgesamt mit seiner Vielzahl Abschnitten beim Expandieren ebenfalls in eine vordefinierte Form gebracht wird und in dieser Form auch verharrt.

Bei der Erfindung ist die Koppeleinrichtung mit einem männlichen und einem weiblichen Steckelement gestaltet, von denen insbesondere eines an einem ersten der beiden Abschnitte und das andere an dem zweiten Abschnitt im wesentlichen ortsfest angebracht ist. Die Steckelemente bilden eine kraft- und/oder formschlüssige Verbindung mittels, der die Abschnitte im komprimierten Zustand des Stents relativ zueinander fixiert werden. Eine weitere Expansion oder Kompression würde die vorgesehenen Steckelemente deformieren. Die Steckelemente sind daher gerade derart steif gestaltet, dass eine gewisse Restflexibilität des Stents im expandierten Zustand erhalten bleibt, dass aber dennoch die gewünschte Formgenauigkeit in diesem expandierten Zustand erreicht wird.

Die Koppeleinrichtung ist mit zwei Steghälften gebildet, die im wesentlichen koaxial ausgerichtet sind und einander gegenüberliegende Endbereiche aufweisen, an denen an einem ersten Endbereich das männliche und an dem zweiten Endbereich das weibliche Steckelement ausgebildet ist. Die Steghälften können bei der Herstellung des erfindungsgemäßen Stents aus einem einzelnen Steg ausgeschnitten und dabei kann an ihnen auch das männliche sowie das weibliche Steckelement ausgebildet werden.

Damit die in der Regel große Anzahl einzelner Koppeleinrichtungen eines erfindungsgemäßen Stents beim Expandieren des Stents alle störungsfrei geschlossen werden können, ist die einzelne Koppeleinrichtung mit einem Führungselement gestaltet, mit dem das männliche Steckelement in das weibliche Steckelement geführt wird, wenn die beiden Abschnitte aufeinander zu bewegt werden.

Die Koppeleinrichtung ist ferner mit zwei Steghälften gebildet, die im wesentlichen koaxial ausgerichtet sind und an einander gegenüberliegenden Endbereichen als Führungselement zum einen einen Führungsstab und zum anderen eine Führungsnut aufweisen. Der Führungsstab und die Führungsnut lassen sich besonders kostengünstig während der Herstellung von Stützstegen der Stentstruktur aus dem Material des Stents (z.B. ein Formgedächtniswerkstoff, insbesondere Nitinol) beispielsweise durch einen Laserschweißvorgang ausschneiden. Die Achse der beiden Steghälften kann sich vorteilhaft im wesentlichen parallel zur Längsachse des Stents erstrecken, denn auf diese Weise kann vorteilhafterweise sichergestellt werden, dass während der Expansion des Stents der Führungsstab nahezu nicht gebogen, sondern nur zusammen mit der Führungsnut parallel versetzt wird. Der Führungsstab tritt daher auch aus der Führungsnut radial nicht heraus.

Damit eine unerwünschte Deformation des erfindungsgemäßen Stents und insbesondere von dessen Koppeleinrichtungen zuverlässig verhindert ist, sind jeweils mit dem Führungselement die beiden Abschnitte bereits im nicht expandierten Zustand des Stents relativ zueinander geführt.

Als formschlüssige Koppelung der Steghälften eines erfindungsgemäßen Stents kann die Koppeleinrichtung mit mindestens einem Hakenelement bzw. Nut/Federelement gestaltet sein, das beispielsweise an einem zugehörigen weiblichen Steckelement verrastet, wenn die beiden Abschnitte aufeinander zu bewegt werden.

Damit bei dem erfindungsgemäßen Stent sowohl die Stentstruktur als auch die darin vorgesehenen Koppeleinrichtungen in nur einem Arbeitsgang aus dem Material des Stents ausgeschnitten werden können, sind vorzugsweise als einzelnes Strukturelement der Stentstruktur mindestens vier Stege vorgesehen, die an Knoten schwenkbar bzw. biegbar miteinander verbunden sind und im expandierten Zustand des Stents eine geschlossene (im wesentlichen polygonartige) Ring-Form, insbesondere eine Rautenform bilden, in deren Inneren die Koppeleinrichtung angeordnet ist. Darüber hinaus kann die Koppeleinrichtung bei dieser Ausgestaltung mittels zweier Steghälften gebildet sein, die im wesentlichen eine Diagonale innerhalb der Ringform bilden.

Hinsichtlich dieser besonders bevorzugten Struktur des erfindungsgemäßen Stents kann die Koppeleinrichtung ferner mit zwei einander gegenüberliegenden Steghälften gebildet sein, die jeweils einzeln zusammen mit zwei benachbarten Stützstegen an einem Knoten der Stentstruktur angelenkt sind.

Gemäß der Erfindung wird weiterhin ein Träger für eine Herzklappe vorgeschlagen, der einen derartigen Stent aufweist.

Gemäß der Erfindung wird weiterhin einTräger für eine Venenklappe, einen Vena Cava Filter, einen prostatischen Sphinkterkörper und/oder als Antireflux Stent (Magenventil) vorgeschlagen, der bzw. die einen derartigen Stent aufweist.

Nachfolgend wird ein Ausführungsbeispiel eines erfindungsgemäßen Stents anhand der beigefügten schematischen Zeichnungen beispielsweise näher erläutert. Es zeigt:
- Fig. 1: eine Detailansicht eines Strukturelements eines erfindungsgemäßen Stents im komprimierten Zustand, bei dem eine Koppeleinrichtung in axialer Richtung des Stents angeordnet ist,
- Fig. 2: die Detailansicht gemäß Fig. 1 im expandierten Zustand,
- Fig. 3: eine Abwicklung einer Umfangreihe von Strukturelementen eines erfindungsgemäßen Stents im komprimierten Zustand und
- Fig. 4: die Abwicklung gemäß Fig. 3 im expandierten Zustand,
- Fig. 5: eine zweite Koppeleinrichtung die in Umfangsrichtung des Stents angeordnet ist,
- Fig. 6: die in Fig. 5 gezeigte Koppeleinrichtung,
- Fig. 7: einen weiteren Stent mit einem nicht kreisförmigen Querschnitt.

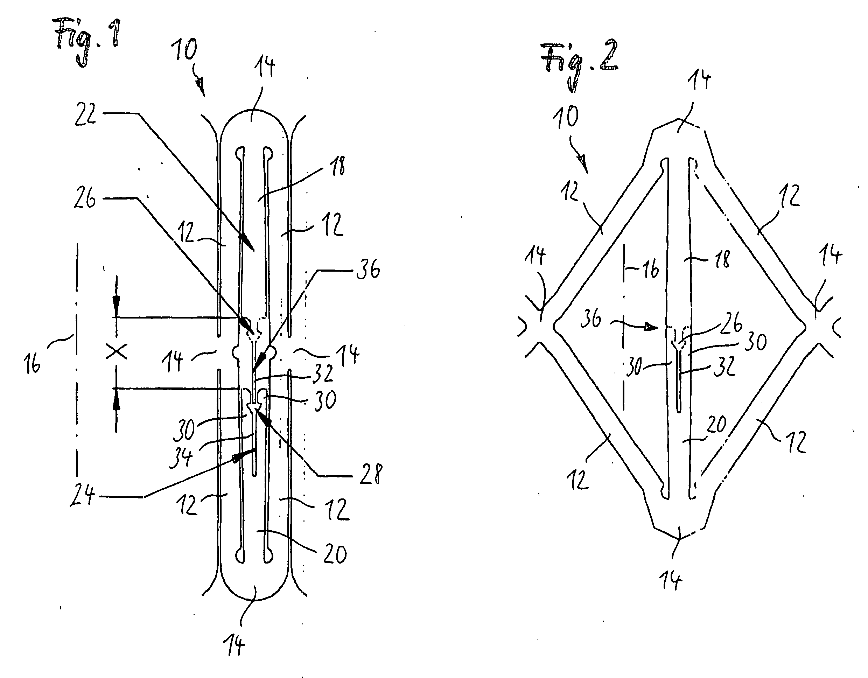

In den Fig. 1 bis 4 ist abschnittsweise ein insgesamt im wesentlichen kreiszylindrischer Stent bzw. Spreiz- bzw. Trägerstruktur 10 zum Einsetzen bzw. Einführen und/oder Expandieren bzw. Stützen bzw. Tragen in einem Lumen dargestellt, dessen Stentstruktur aus einer Vielzahl von Stützstegen 12 zusammengesetzt ist. Die Stützstege 12 enden jeweils an Knoten 14, die als Verbindungsabschnitte für die Stützstege 12 dienen.

Die Stützstege 12 erstrecken sich im komprimierten Zustand des Stents 10 im wesentlichen in Richtung einer Längsachse 16 des Stents 10 und bilden dabei eine Art flachgedrückte Raute, in der im Inneren zwei koaxial angeordnete Steghälften 18 und 20 angeordnet sind. Die Steghälften 18 und 20 sind an ihren voneinander abgewandten Enden jeweils an einem der Knoten 14 angelenkt und weisen an ihren zueinander gewandten Endbereichen ein männliches bzw. ein weibliches Steckelement 22 und 24 auf.

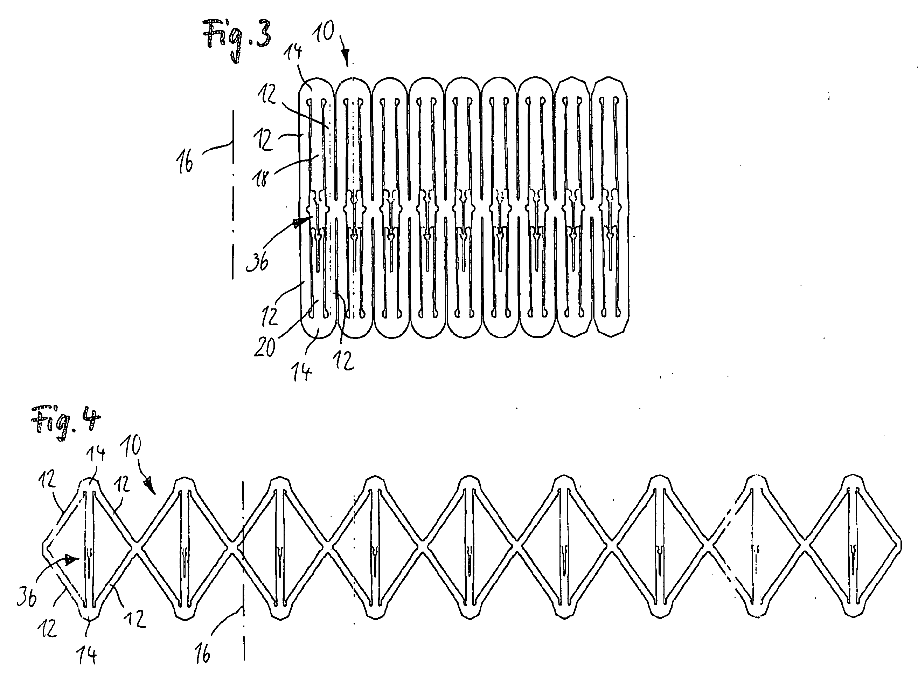

An dem männlichen und dem weiblichen Steckelement 22 bzw. 24 sind jeweils einander gegenüberliegende Anlageflächen ausgebildet, die in dem in Fig. 1 und 3 veranschaulichten komprimierten Zustand des Stents 10 einen definierten Abstand bzw. Wegstrecke X voneinander entfernt angeordnet sind und erst beim Expandieren des Stents 10 durch die Bewegung der zugehörigen Knoten 14 aufeinander zu bewegt werden.

Bei dieser Bewegung wird die aus dem männlichen und dem weiblichen Steckelement 22 bzw. 24 gebildete Steck- bzw. Schnappverbindung zusammengeführt und in dieser Stellung verrastet. Das Verrasten geschieht mittels einer oder mehreren Rastnasen 26, die an dem männlichen Steckelement vorgesehen sind und die jeweils an einer entsprechenden Aussparung 28 des weiblichen Steckelements 24 einrasten. Damit die Rastnasen 26 in das weibliche Steckelement 24 eingeführt werden können, ist dieses mittels zweier Federarme 30 gestaltet, in denen die Aussparungen 28 ausgebildet sind. Die Rastnase(n) 26 ist bzw. sind bevorzugt haken- bzw. doppelhaken- bzw. pfeilförmig während die entsprechende(n) Aussparung(en) 28 eine im wesentlichen komplementäre Form aufweisen.

Bei einem selbstexpandierenden Stent 10 sollte dabei die Expansionskraft größer als die Gegenkraft dieser Federarme 30 gegen die Rastnasen 26 sein. Um dies zu erreichen, sollte der letzte Schritt einer Wärmebehandlung eines solchen Stents die Differenz vom Ansetzen des Stents bis zum Einrasten von dessen Schnappverbindungen überbrücken. Ferner kann die Austenit-finish-Temperatur (Af-Temperatur) des Stentmaterials so eingestellt sein, dass das Handhaben des Stents 10 bei normaler Umgebungstemperatur, d.h. einer Temperatur von zirka 20 ° C bis 25 ° C, möglich ist. Fremdgesteuert expandierende Stents 10 werden erst bei der Anwendung des Stents manuell bzw. gesondert expandiert. Bei solches Stents 10 muss sichergestellt sein, dass die Federarme 30 an den Schnappverbindungen beim Expandieren nicht dauerhaft bzw. plastisch verformt werden.

An dem männlichen Steckelement 22 ist ferner im vordersten Bereich ein Führungsstab 32 ausgebildet, der bereits im komprimierten Zustand des Stents 10 in einer Führungsnut 34 des weiblichen Steckelements 24 geführt und darin weiter verschiebbar ist.

Im verrasteten Zustand des männlichen und des weiblichen Steckelements 22 bzw. 24 liegen die zugehörigen, oben genannten Anlageflächen aneinander an und es ist insgesamt eine verrastete Koppeleinrichtung 36 geschaffen. Mit dieser Koppeleinrichtung 36 sind die ansonsten beweglichen, zu den Steghälften 18 und 20 gehörenden Knoten 14 relativ zueinander fixiert und die Stützstege 12 sind in einer im wesentlichen nicht mehr verformbare Rautenform arretiert.

Die vielen in Umfangsrichtung des Stents 10 nebeneinander angeordneten Rauten, wie sie in Fig. 4 veranschaulicht sind, führen beim Expandieren des Stents 10 zu einer Vergrößerung des Umfangs des Stents 10 und damit zu der gewünschten Vorgabe einer definierten Strömungsquerschnittsfläche in dem Lumen.

Da bevorzugt in jeder einzelnen Raute jeweils eine Koppeleinrichtung 36 ein weiteres Verformen der Rautenform verhindert ist die expandierte Gesamtform des Stents 10 fixiert und kann im wesentlichen nicht mehr verändert werden. Eine undefinierte weitere Expansion und/oder Kompression des Stents 10 ist damit sicher verhindert. Es ist jedoch denkbar, Koppeleinrichtungen 36 nur in jeder zweiten oder weiteren Rautenform (bevorzugt im wesentlichen in Umfangsrichtung des Stents regelmäßig) vorzusehen.

Es ist weiterhin denkbar, daß entlang der Bewegungsrichtung der Knoten 14 zueinander eine Vielzahl von Rastmöglichkeiten vorgesehen ist, z.B. durch Hintereinander-Vorsehen einer Vielzahl von Rastnasen 26 und/oder entsprechenden Aussparungen 28, so daß der Stent 10 in verschiedenen Expansionsgraden bzw. - zuständen expandiert angeordnet sein kann. In anderen Worten kann durch ein Vorsehen von Kopplungseinrichtungen an verschiedenen axialen Positionen bzw. Expansionsgraden sichergestellt werden, daß der Stent bevorzugt in verschiedenen Expansionszuständen (in denen die Knoten um verschiedene Wegstrecken X1...Xn zueinander hin bewegt sind) verrastet werden kann.

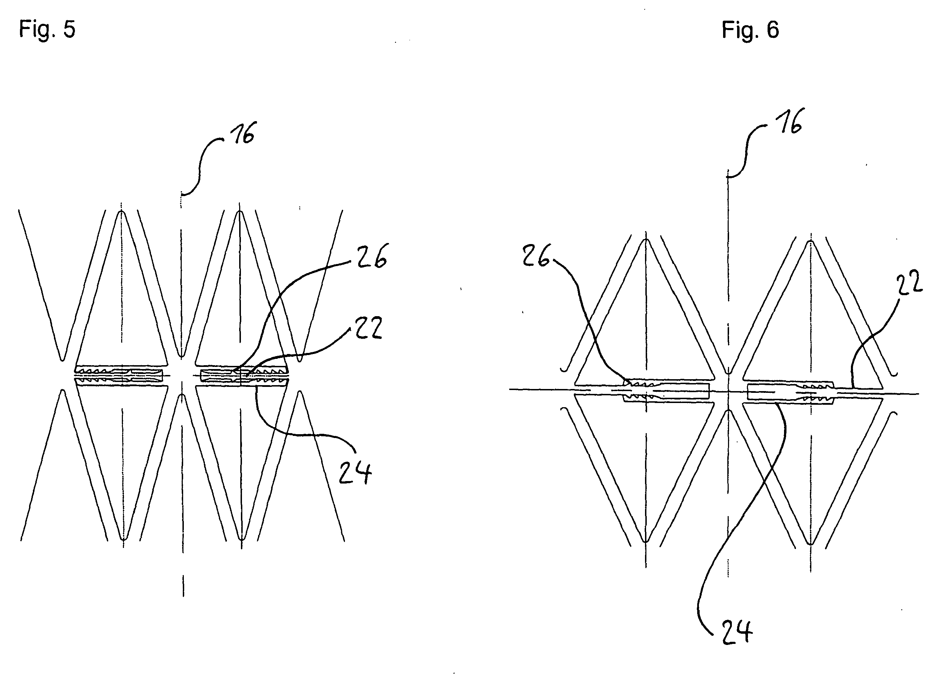

In den Figuren 5 und 6 ist eine zweite Koppeleinrichtung gezeigt, bei der die Steckelemente 22, 24 in Umfangsrichtung des Stents 10, d.h. im wesentlichen rechtwinklig zu der Längsachse 16 des Stents 10 vorgesehen sind. Bei einer derartigen Koppeleinrichtung werden die Steckelemente 22, 24 bei der Expansion des Stents 10 voneinander weg bewegt, wie in Fig. 6 gezeigt ist. In anderen Worten befinden sich die Steckelemente 22, 24 bei diesem Stent 10 im komprimierten Zustand des Stents 10 in der angenäherten Lage, wie in Fig. 5 gezeigt ist, und bewegen sich voneinander weg, wenn der Stent 10 expandiert wird, wie in Fig. 6 gezeigt ist.

Demgemäß sind die Rastnase(n) 26 des mänlichen Steckelements 22 und die Aussparung(en) 28 des weiblichen Steckelements 24 so ausgebildet, dass eine Verrastung im voneinander weg bewegten Zustand erfolgt. Dabei kann bei der Ausbildung mehrerer Rastnasen 26 und/oder Aussparungen 28 eine mehrstufige Verrastung gebildet werden.

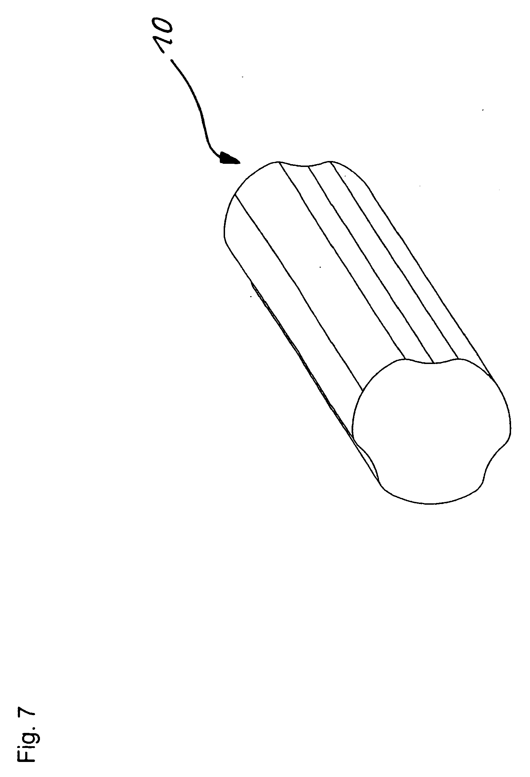

Es können auch die Steckelemente 22, 24 sowohl in Umfangsrichtung als auch in axialer Richtung in Kombination eingesetzt werden. Außerdem können durch unterschiedliche Ausbildung der Steckelemente von einer Kreisform abweichende Formen eines expandierten Stents 10 gebildet werden, wie in Fig. 7 gezeigt ist.

Der in Fig. 7 gezeigten Stent 10 weist drei Einschnürungen an seinem Umfang im expandierten Zustand auf, die durch Ausbilden unterschiedlicher Steckelemente 22, 24 gebildet werden. Es können jedoch auch andere Formen des expandierten Stents 10 durch unterschiedliche Steckelemente 22, 24 gebildet werden, wie beispielsweise eine Elipsenform, eine Konusform, eine Wellenform etc.

Die Herstellung des Stents gemäß der Erfindung oder eine bevorzugte Ausführungsform hiervon kann sowohl aus Rohmaterial als auch aus Flachmaterial erfolgen wobei bei letzterem der Stent später gerollt, geschweißt und/oder endbearbeitet wird. Weiterhin kann die Herstellung des Stents mittels Laserschneiden, Laserabtrag, photochemisches Ätzen und/oder Erodieren erfolgen. Weiterhin kann die Herstellung des Stents auch derart erfolgen, daß die Stentstruktur in einer zumindest teilweise expandierten Form gefertigt wird und der Stent anschließend zum Einführen z.B. in den Katheter auf eine komprimierte Form verkleinert wird, bevor er nachfolgend im Körper wieder zumindest teilweise expandiert wird.

Die Erfindung kann besonders sinnvoll bei ballonexpandierten Stents aus rostfreiem Edelstahl, Tantal, Niob, Kobaltlegierungen und anderen Werkstoffen wie z.B. Polymeren, selbstabbaubaren Werkstoffen (z.B. Milchsäure-Werkstoffen bzw. - Derivate), sowie bei Stents aus Nitinol (Nickel-Titan-Legierungen) und/oder aus anderen selbstexpandierbaren Werkstoffen bzw. Formgedächniswerkstoffen verwendet werden.

Besonders bevorzugt wird der erfindungsgemäße Stent bzw. Spreiz- bzw. Trägerstruktur verwendet zum Stabilisieren von Gefäßen, insbesondere von Blutgefäßen oder als Trachealstent, Bronchial-Stent, transhepatischer Portosytemischer Shunt, Transhepatischer Intravenöser Portosytemischer Shunt (TIPS), Gallengang-Stent und/oder Schutzvorrichtung gegen Embolien (Englisch: Embolic Protective Device). Weiterhin bevorzugt wird der erfindungsgemäße Stent bzw. Spreiz- bzw. Trägerstruktur verwendet als Träger-Stent für Implantate insbesondere für eine Herzklappe, eine Venenklappe, einen Vena Cava Filter, einen prostatischen Sphinkterkörper und/oder als Antireflux Stent (Magenventil).

### Bezugszeichenliste

- 10: Stent
- 12: Stützsteg
- 14: Knoten
- 16: Längsachse
- 18: Steghälfte
- 20: Steghälfte
- 22: männliches Steckelement
- 24: weibliches Steckelement
- 26: Rastnase
- 28: Aussparung
- 30: Federarm
- 32: Führungsstab
- 34: Führungsnut
- 36: Koppeleinrichtung
- X: Abstand zwischen Anlageflächen

## Patentansprüche

1. Stent (10) zum Einsetzen und/oder Expandieren in einem Lumen, mit minde-stens zwei Abschnitten (14) einer Stentstruktur, die beim Expandieren des Stents (10) aufeinander zu bewegt werden,
wobei zwischen den beiden Abschnitten (14) mindestens eine Koppeleinrichtung (36) angeordnet ist, mittels der nach zumindest einer vordefinierten Bewegung (X) der beiden Abschnitte (14) diese Abschnitte (14) miteinander gekoppelt werden,
wobei ein Entfernen der beiden Abschnitte (14) weg voneinander durch die Koppeleinrichtung (36) verhindert ist, wenn die Koppeleinrichtung (36) nach der vordefinierten Bewegung (X) der beiden Abschnitte (14) diese Abschnitte (14) miteinander koppeit, so daß die expandierte Gesamtform des Stents (10) derart fixiert ist, daß sie nicht mehr verändert werden kann,
Wobei, die Koppeleinrichtung (36) mit zwei Steghälften (18; 20) gebildet ist die koaxial ausgerichtet sind und an einander gegenüberliegenden Endbereichen ein männ liches und ein weibliches Stechelement (22, 24) aufereisen, wobei an dem männlishen Stechelement (22) eine order mehrere Rastnasen (26) rorgosehen sind Undan dem Weiblichen Stechelement (24) eine oder mehrereentsprachende Hassparungen (28) vorge- sehen sind, daduch gekennzeichenet, dass an dem mànnlichen Stechelement (22) im vordersten ßereich ein Führungsstabt (32) ausgebildet ist, wobei der Führungsstab (32) bereits im komprimierten Zustand des Stents (10) in einer Führungsnut (34) des weiblichen Stechelements (24) geführt und darin weiter verschiebbar ist.

2. Stent (10) nach Anspruch 1, des weiteren mit mindestens zwei Abschnitten einer Stentstruktur, die beim Expandieren des Stents (10) voneinander weg bewegt werden,
wobei zwischen den beiden Abschnitten mindestens eine zweite Koppeleinrichtung angeordnet ist, mittels der nach zumindest einer vordefinierten Bewegung (X) der beiden Abschnitte diese Abschnitte miteinander gekoppelt werden, derart, dass ein Bewegen der beiden Abschnitte aufeinander zu verhindert ist.

3. Stent nach Anspruch 1 oder 2, wobei eines aus dem männlichen und dem weiblichen Steckelement (22; 24) an einem ersten der beiden Abschnitte (14) und das andere an dem zweiten Abschnitt (14) ortsfest angebracht ist.

4. Stent nach einem der vorherigen Ansprüche, wobei mindestens vier Stützstege (12) vorgesehen sind, die an Knoten (14) schwenkbar miteinander verbunden sind und im expandierten Zustand des Stents (10) eine geschlossene Ring-Form bilden, in deren Inneren die Koppeleinrichtung (36) angeordnet ist.

5. Stent nach Anspruch 4, wobei die geschlossene Ring-Form eine Rautenform bildet.

6. Stent nach einem der vorherigen Ansprüche, wobei jede Steghälfte 18; 20) an zwei benachbarten Stützstegen (12) an einem Knoten (14) der Stentstruktur angelenkt ist.

7. Stent nach einem der vorherigen Ansprüche, wobei die Koppeleinrichtung (36) sich im wesentlichen in Richtung einer Längsachse (16) des Stents (10) erstreckt.

8. Stent nach einem der Ansprüche 2 bis 6, wobei die zweite Koppeleinrichtung sich im wesentlichen in Umfangsrichtung des Stents (10) erstreckt.

9. Träger für eine Herzklappe mit zumindest einem Stent gemäß einem der vorangehenden Ansprüche.

10. Träger für eine Venenklappe, Vena Cava Filter, prostatischer Sphinkterkörper und/oder Antireflux-Stent mit zumindest einem Stent gemäß einem der vorangehenden Ansprüche.

## Claims

1. A stent (10) for insertion and/or expansion in a lumen, with at least two portions (14) of a stent structure, which upon expansion of the stent (10) are moved toward each other,
wherein at least one coupling device (36) is arranged between the two por tions (14), by means of which the portions (14) are coupled to each other after at least one predefined movement (X) of the two portions (14),
wherein a removal of the two portions (14) away from each other is prevented by the coupling device (36) when the coupling device (36) couples the portions (14) to each other after the predefined movement (X) of the two portions (14), so that the expanded overall form of the stent (10) is fixed such that it cannot be changed any more,
wherein the coupling device (36) is formed with two web halves (18; 20) coaxially aligned and having male and female connection elements (22, 24) at mutually opposite end regions thereof, one or more latching noses (26) being provided on the male connection element (22) and one or more corresponding recesses (28) being provided on the female connection element (24), **characterized in that** a guide rod (32) is formed on the male connection element (22) in the frontmost region thereof, the guide rod (32) being guided in a guide groove (34) of the female connection element (24) and further slidable therein already in a compressed state of the stent (10).

2. The stent (10) according to claim 1, further with at least portions of a stent structure, which are moved away from each other upon expansion of the stent (10),
wherein at least one second coupling device is arranged between the two portions, by means of which the portions are coupled to each other after at least one predefined movement (X) of the two portions such that a movement of the two portions toward each other is prevented.

3. The stent according to claim 1 or 2, wherein one of the male and female connection elements (22; 24) is stationarily attached to a first one of the two portions (14) and the other to the second portion (14).

4. The stent according to one of the preceding claims, wherein at least four support webs (12) are provided, which are pivotally connected to each other at knots (14) and form a closed ring shape in the expanded state of the stent (10), in the interior of which the coupling device (36) is arranged.

5. The stent according to claim 4, wherein the closed ring shape forms a lozenge shape.

6. The stent according to one of the preceding claims, wherein each web half (18; 20) is hinged to two adjacent support webs (12) at a knot (14) of the stent structure.

7. The stent according to one of the preceding claims, wherein the coupling device (36) substantially extends in the direction of a longitudinal axis (16) of the stent (10).

8. The stent according to one of claims 2 to 6, wherein the second coupling device substantially extends in the circumferential direction of the stent (10).

9. A carrier for a heart valve, with at least one stent according to one of the preceding claims.

10. A carrier for a venous valve, vena cava filter, prostatic sphincter body and/or antireflux stent, with at least one stent according to one of the preceding claims.

## Revendications

1. Stent (10) destiné à être introduit et/ou expansé dans une lumière, avec au moins deux tronçons (14) d'une structure de stent qui peuvent être déplacés l'un vers l'autre lors de l'expansion du stent (10),
au moins un dispositif de couplage (36) étant disposé entre les deux tronçons (14), au moyen duquel, après au moins un mouvement (X) prédéfini des deux tronçons (14), ces tronçons (14) sont couplés l'un à l'autre,
un éloignement des deux tronçons (14) l'un de l'autre étant empêché par le dispositif de couplage (36) quand le dispositif de couplage (36), après le mouvement (X) prédéfini des deux tronçons (14), couple ces tronçons (14) l'un à l'autre de sorte que la forme globale expansée du stent (10) est fixée de sorte qu'elle ne peut plus être modifiée,
le dispositif de couplage (36) étant formé avec deux moitiés d'entretoise (18 ; 20) qui sont orientées de façon coaxiale et qui présentent, au niveau de zones d'extrémité se faisant face l'une l'autre, un élément de connexion mâle (22) et un élément de connexion femelle (24), un ou plusieurs becs d'encliquetage (26) étant prévus sur l'élément de connexion mâle (22), et un ou plusieurs évidements (28) correspondants étant prévus sur l'élément de connexion femelle (24), **caractérisé en ce que**, sur l'élément de connexion mâle (22), il est constitué dans la zone la plus en avant une tige de guidage (32), la tige de guidage (32) étant, déjà dans l'état comprimé du stent (10), guidée dans une rainure de guidage (34) de l'élément de connexion femelle (24) et pouvant y être déplacée davantage intérieurement.

2. Stent (10) selon la revendication 1, en outre avec au moins deux tronçons d'une structure de stent qui, lors de l'expansion du stent (10), sont éloignés l'un de l'autre, au moins un deuxième dispositif de couplage étant disposé entre les deux tronçons, dispositif de couplage au moyen duquel, après un mouvement (X) prédéfini des deux tronçons, ces tronçons sont couplés l'un à l'autre de sorte qu'un déplacement des deux tronçons l'un vers l'autre est empêché.

3. Stent selon la revendication 1 ou 2, un élément de connexion parmi l'élément de connexion mâle (22) et l'élément de connexion femelle (24) étant mis en place de façon fixe sur un premier des deux tronçons (14), et l'autre élément de connexion étant mis en place de façon fixe sur le deuxième tronçon (14).

4. Stent selon une des revendications précédentes, au moins quatre entretoises d'appui (12) étant prévues qui sont raccordées les unes aux autres en pivotement au niveau de noeuds (14) et forment, dans l'état expansé du stent (10), une forme d'anneau fermée à l'intérieur de laquelle est disposé le dispositif de couplage (36).

5. Stent selon la revendication 4, la forme d'anneau fermée formant une forme de losange.

6. Stent selon une des revendications précédentes, chaque moitié d'entretoise (18 ; 20) étant articulée au niveau de deux entretoises d'appui (12) voisines au niveau d'un noeud (14) de la structure de stent.

7. Stent selon une des revendications précédentes, le dispositif de couplage (36) s'étendant essentiellement en direction d'un axe longitudinal (16) du stent (10).

8. Stent selon une des revendications 2 à 6, le deuxième dispositif de couplage s'étendant essentiellement dans la direction circonférentielle du stent (10).

9. Support pour une valvule cardiaque avec au moins un stent selon une des revendications précédentes.

10. Support pour une valvule veineuse, filtre de veine cave, corps de sphincter prostatique et/ou stent anti-reflux avec au moins un stent selon une des revendications précédentes.
